**Europäisches Patentamt**

**European Patent Office**

**Office européen des brevets**

(19)

(11) Publication number : **0 551 762 A1**

# (12) EUROPEAN PATENT APPLICATION

(21) Application number : **92311772.5**

(22) Date of filing : **23.12.92**

(51) Int. Cl.$^5$ : **C08K 11/00**, C08L 99/00

(30) Priority : **26.12.91 JP 344271/91**
**28.02.92 JP 43740/92**

(43) Date of publication of application :
**21.07.93 Bulletin 93/29**

(84) Designated Contracting States :
**BE CH DE FR GB IT LI NL SE**

(71) Applicant : **IDEMITSU PETROCHEMICAL CO. LTD.**
**1-1, Marunouchi 3-chome Chiyoda-ku Tokyo 100 (JP)**

(72) Inventor : **Sano, Masahiro, c/o Idemitsu Petrochem. Co., Ltd.**
**841-3, Kou, Shirahama-cho Himeji-shi, Hyogo-ken (JP)**
Inventor : **Ohyama, Shigeru, c/o Idemitsu Petrochem. Co., Ltd.**
**841-3, Kou, Shirahama-cho Himeji-shi, Hyogo-ken (JP)**

(74) Representative : **Jackson, Peter Arthur GILL JENNINGS & EVERY, Broadgate House, 7 Eldon Street London EC2M 7LH (GB)**

(54) Composition containing powder or fibres of natural sponge.

(57)    A sponge-containing composition, which comprises such a base material as a synthetic resin or rubber and contains at least one sponge material selected from the group consisting of sponge powder with an average grain size of, for instance, 0.1 to 100 $\mu$m and sponge fibers with a fiber length of, for instance, 1 cm or below, the sponge material being bleached if necessary. A product such as films, sheets, laminates, coatings, ink, fibers and processed fiber materials obtained from the same sponge-containing composition. A sponge containing product containing at least one sponge material selected from the group noted above, the sponge material or materials being attached to such a base material as fibers.

EP 0 551 762 A1

This invention relates to sponge-containing compositions, sponge-containing products obtained from the same compositions and sponge-containing products, having applications to resin moldings, rubber products, films, sheets, synthetic or artificial leather, paints, ink, cosmetics, textiles, clothing, diaper covers, sanitary products, interior materials, construction materials, bathing sponges, cosmetic puffs, poultices, emergency plasters, freshness-retaining bags, paper towels, food additives, medical products, diaper sheets, pads, consumable surgical operation garments, gloves, fishing nets, vinyl house films and various other fields.

In the prior art, sponge is treated with a potassium permanganate solution, a blend solution of sulfuric acid and sodium thiosulfate and an ammonia solution in the mentioned order to obtain yellow sponge. It is difficult to make sponge white, whereas sponge having a high white degree is desired for various applications.

It has been difficult to finely pulverize sponge. At present, therefore, no composition is available, which contains powdery or fibrous sponge. Up to date, sponge has been utilized only as such or in cut forms of suitable sizes for cosmetics and office business items.

In the meantime, there are various quantity improvement demands in the fields related to the living. For example, regarding clothing, vehicle interior materials and construction materials, there are demands for improvements of the appearance and touch and also improvements of such functions as moisture absorption. As a well-known additive to artificial materials to meed the above demands, there is natural cattle hide fiber powder. For the natural cattle hide fiber powder to withstand actual use, it has been necessary to improve the thermal properties of the fiber powder by introducing cross-links with a tanning or like treatment.

The tanning treatment, however, is not only cumbersome in operation but also poses safety problems due to residual chemicals.

An object of the invention is to provide sponge in the form of powder and also in the form of fibers having excellent whiteness degree and also provide sponge-containing compositions and products comprising such sponge-containing compositions, which are free from the above safety problems, can be provided inexpensively and permit improvement of the appearance and various functions such as moisture absorption.

According to the invention, there are provided (I) sponge-containing compositions containing powdery or fibrous sponge introduced into synthetic resins, rubber, cosmetic materials, etc., (II) films, sheets, laminates of such films and sheets, paints, ink and fibers produced from the compositions noted above, and (III) sponge-containing products obtained by attaching powdery or fibrous sponge to textiles, woven cloths, resin sheets, wood, metal sheets, etc.

Now, sponge materials, resins, rubber and so forth used according to the invention will be described.

## (1) Materials of powdery and fibrous sponge

As the materials of the powdery and fibrous sponge may be used various kinds of sponge.

In addition to these varieties of sponge which can be used as the material of the sponge in the form of powder and so forth according to the invention, it is also possible to use sponge cutting dust resulting when the sponge is processed to obtain cosmetics, bathing sponge, fountain pen tip ink absorber, etc. Such sponge cutting dust heretofore could have found practically no application. According to the invention, it can be effectively utilized as material.

The powdery sponge according to the invention can be produced by pulverizing the materials noted above in various processes.

## (I) Pulverization process 1

First, sponge is cut into chips, which are then suitably coarsely crushed prior to regular pulverization. Cutting dust may be directly coarsely pulverized. The coarse crushing is suitably carried out by using such mills as cutter mils and frictional mills or such compression crushers as jaw crushers and roll crushers.

The coarsely crushed sponge thus obtained is then pulverized by dry or wet pulverization by using vibration mills, ball mills, high speed rotational impact pulverizers, high speed rotational shearing pulverizers, stirring pulverizers and so forth.

The pulverizing conditions, and drying conditions in the case of the wet pulverization, are suitably selected depending on the material used and desired grain size of powdery sponge.

For uniformalizing the grain size of the powdery sponge thus obtained, it is suitable to carry out a repeated process of shieving out coarse particles and pulverizing again the coarse particles in the above manner or by using an air stream pulverizer or the like.

## (2) Pulverization process 2

Sponge is dissolved in an alkali solution. Ready dissolution can be obtained by preliminarily cutting the sponge into chips or reducing the grain diameter by coarse crushing. As the alkali solution may be used a sodium hydroxide solution, a potassium hydroxide solution, etc.

Subsequently, the solution obtained by the dissolution is jet into gas or like, thus effecting pulverization. The gas is air or an inert gas, and the liquid is an organic solvent, e.g., methanol and ethanol, or water, brine or like aquaous solution. The powdery sponge obtained in this way is suitably pulverized again by using an air stream pulverizer or the like.

The grain size of the powdery sponge is desirably smaller from the standpoint of molding the composition, but it is desirably greater for obtaining better appearance of the natural material, and incorporation of short fibers can improve the touch and design properties. When its use is considered, the powdery sponge according to the invention usually suitably has an average grain size of 0.1 to 100 μm, preferably 0.1 to 60 μm. It is practically difficult to obtain an average grain size below 0.1 μm. On the other, if the the average grain size exceeds 100 μm, it leads to such problems as the generation of a rough feel or reduction of the mechanical strength of the compositions obtainable by incorporating the powdery sponge into various materials. However, in applications to localities not touched by the man or the like with expectation of moisture absorption, anti-bacteria, ultra-violet cutting and other functions, the average grain size may be above 100 μm. This also applies to the average length of fibrous sponge. That is, the average length of the fibrous sponge is suitably 1 cm or below, preferably 0.01 to 5 mm.

Highly white powder or fibrous sponge may be obtained by bleaching in various processes.

The bleaching treatment may be carried out either on the material sponge or on the powdery or fibrous sponge. When the material sponge is bleached, the powdery sponge is produced in the above process.

The bleaching may be carried out in an oxidization bleaching process, a reduction bleaching process or a multi-stage bleaching process, in which the oxidization and reduction bleaching processes are combined. As the oxidization bleaching process, there are a hypochlorous acid process, a chlorous acid process, a hydrogen peroxide process, etc., these processes being usually used. Where a chlorine type bleaching agent is used for the oxidization bleaching, a chlorine removal treatment is suitably made after the bleaching. The chlorine removal may be carried out by using an aquaous chlorine removal agent solution such as a sodium thiosulfate solution or a sodium sulfite solution. Alternatively, bleaching with hydrogen peroxide may be carried out subsequently.

The reduction bleaching may be carried out in a process using hydroxymethane sulfinic acid salt as principal component or in a process using a sulfite type reduction bleaching agent, e.g., sulfite gas, acidic sodium sulfite and sodium hyposulfite.

It is possible to make the material sponge or powdery sponge sufficiently white with the sole oxidization or reduction bleaching. For while color stability improvement, however, the bleaching is suitably carried out by a multi-step bleaching process, in which the oxidization bleaching and reduction bleaching are combined. Specifically, there is a three-step process. A first step is the oxidization bleaching with a chlorine type bleaching agent, such as the hyposulfite or sulfite process, followed by the chlorine removal with an aqueous chlorine removal agent solution with sodium thiosulfate or sodium hyposulfite. A second step is the hydrogen peroxide bleaching. A third step is reduction bleaching with hydroxymethane sulfinic acid salt. With this process, it is possible that the powdery sponge is stably held white without possibility of becoming yellow.

The conditions for the bleaching process may be suitably selected. However, the bleaching temperature is usually as high as 80 to 90 °C. However, when sponge is bleached at a temperature of 60 °C or above, it becomes brawn. Therefore, the bleaching is carried out at 50 °C or below, preferably 4 to 40 °C.

The white powdery or fibrous sponge obtained after the above bleaching according to the invention, has a whiteness degree of 30 or above, preferably 70 or above.

The whiteness degree W is measured using a color difference meter ("CR-200" manufactured by Minoruta Camera), and its numerical value is obtained by using the following equatioN (I).

$$W = 100 - ((100 - L)2 + K2(a - ap)2 + (b - bp)1/2 \quad (I)$$

ap, bp:      chromatic NESU exponents in a standard white L, a, b system, with value of 0.00
K2:      a constant, being 1 in general
L, a, b:      numerical values as measurements of the color difference meter

The powdery or fibrous sponge according to the invention is bacteria-resistant and rust-proof. It may be incorporated by 1 to 90 %, preferably 1 to 60 %, by weight. If the amount incorporated is less than 1 % by weight, the effects of incorporation of the powdery or fibrous sponge can not be obtained. Increasing the amount has an effect of improving the moisture absorption property and sense of touch of the composition, but reduces the mechanical strength.

(2) Synthetic resins, rubber, etc.

According to the invention, either thermoplastic or thermosetting synthetic resins may be used.

Examples of the thermoplastic resin which may be used are vinyl chloride resins, vinyl acetate resins, polystyrene, ABS resins, acrylic acid resins, polyethylene, fluorine resins, polyamide resins, acetal resins, polycarbonate, urethane, ester and like type thermoplastic elastomers, and cellulose type plastics.

Examples of the thermosetting resin are phenol resins, urea resins, melamine resins, unsaturated polyester resins, epoxy resins, diallyl phthalate resins, thermosetting polyurethane resins and silicon resins.

Examples of the rubber which may be used according to the invention are synthetic rubber of diene type (e.g., butadiene-styrene, and butadiene-acrylonitrile), polysulfide type (i.e., thioglycol), olefin type (ethylene-propylene, and polyethylene chlorosulfonate), organic silicon compound type (i.e., fluorine-containing compound type), urethane type and vinyl type and also natural rubber.

For such purpose as quality stabilization or depending on the environments of use, the composition according to the invention may incorporate adequate amounts of well-known substances in the art, i.e., (I) such additives as fibers, anti-oxidization substances, ultra-violet absorbers and ultra-violet scattering substances, (II) fillers, (III) processibility improvers, (IV) hardening substances or vulcanizers, (V) dyes, pigments and other coloring substances, and (VI) solvents.

Examples of the fibers to be added are natural fibers such as cotton, linen, silk and fur, and chemical fibers such as layon, cuplar, polyester, polyethylene, polypropylene, polyurethane, vinylon, acryle, nylon, acetate and polyvinyl chloride.

Examples of the anti-oxidization substance are alkylphenol, alkylene bisphenol, alkylphonol thioether, $\beta$, $\beta'$-thirpropionic acid ester, organic phosphoric acid ester, aromatic amine, and phonol-nickel complexes.

Examples of the ultra-violet absorbers are salicylic acid compounds, e.g., phenyl salicylate, azole compounds such as 2-(2-hydroxy-5-methylphenyl) benzotriazole, benzophenone compounds,e.g., 2-hydroxybenzophenone, 2-hydroxy-4-methoxybenzeophenone, 2-hydroxy-4-octoxybenzohenone, 2,2'-dihydroxsy,4-methoxybenzeophone, paraaminobenzoic acid compounds, e.g., ethyl paraaminobenzoate, cinnamic acid compounds, e.g., cinnoxat, and urocanic acid. Examples of the scattering substance are titanium oxide, calion, calcium crbonate and talc.

Examples of the processibility improver for vinyl chloride are such plasticizers as dioctyl phthalate, dibutyl phphalate, tricresyl phosphate and trioctyl phosphate, such stablizers as tribasic lead sulfate, lead stearate and dibasic phosfite, and such lubricants as stearic acid, lead stearate and carnauba wax. Those for polyurethane are such solvents as dimethylformamide, methylethyl ketone and isopropylalcohol. Examples of the filler are calcium carbonite, titanium white, clay and mica. Examples of the vulcanizer are phosphorus, e.g., powdery phosphorus, insluble phosphorus and surface-treated phosphorus, such metal oxides as magnesium oxide, zinc (for CR, CSM and T), quinoid vulcanizers, peroxide vulcanizers, amine vulcanizers, reesin vulcanizers and organic phosphorus vulcanizers. Examples of the vulcanization promoter are diocarbamic acid salt, and complex ring mercaptan.

Examples of the vulcanization promotion aider are zinc flower and stearic acid.

To produce the sponge-containing composition according to the invention, resin and/or rubber in the form of pellets, power, blocks or paste may be used. In this case, to prevent foaming and hydrolysis of powdery and/or fibrous sponge when mixing or kneading the components while heating them, the sponge component is dried, and the dry sponge component is mixed or kneaded with resin and/or rubber, and also with additives if necessary, while these components are heated, the paste thus obtained being processed into pellets or powdery form. When producing the composition in the form of a liquid, resin and/or rubber are dissolved or dispersed in an adequate solvent, then the sponge component and additives, if necessary, are added to the solution or dispersoid thus obtained, and the resultant system is stirred.

In either case, the sponge component is used by 90 to 1 % by weight with respect to 10 to 99 % by weight of the resin and/or rubber. If the proportion of the sponge component exceeds 90 % by weight, the composition becomes fragile. If the composition is less than 1 % by weight, on the other hand, the effects of incorporating the sponge component can not be obtained. Preferred proportion of the sponge component is 1 to 60 % by weight.

(3) Films and sheets

The sponge-containing film or sheet according to the invention may be produced by using synthetic resins in the form of pellets, powder, blocks or paste. In this case, to prevent foaming or hydrolysis of the powdery and/or fibrous sponge component, the sponge component is dried, and the dry sponge component is kneaded together with resin and addives, if necessary, into pellets or powder, for instance. This pellet-like or powdery

4

material is formed into a film or a sheet. It is possible to produce a temporary liquid composition. In this case, the resin is is dissolved or dispersed in an adequate solution, the sponge component is then added together with additives, if necessary, and the system is stirred or kneaded and then formed into a film or a sheet.

In either case, the sponge component is incorporated by 90 to 1 % by weight with respect to 10 to 99 % by weight of the synthetic resin. If the proportion of the sponge component exceeds 90 % by weight, the film or sheet may become fragile. If the proportion is less than 1 % by weight, the effect of adding the sponge can not be obtained. In the case of the film, the content of the sponge component in the synthetic resin is suitably 1 to 50 % by weigh, whereas in the case of the sheet it is suitably 1 to 60 % by weight. The adequate thickness of the film or sheet is 5 $\mu$m or above, and the sponge component is suitably uniformly dispersed. The sponge component may be exposed to the surface of the film or sheet. In this case, the touch and sense or warmness may further be improved. The film or sheet may be formed in a usual manner, for instance a dry film formation process, a wet film formation process, a calender process, an inflation process, a T die molding process and a press molding process.

The base material used for producing the sponge-containing film or sheet according to the invention is not limited to synthetic resins, but it is possible to use rubber as well.

(4) Laminates

While the sponge-containing film or sheet as noted in (3) may be used by itself, it is also possible to laminate the film or sheet on a base material for various applications. Further, if necessary, the surface may be finished by buffing or embossing roll processing. As the base material may be used woven, braided and non-woven cloths of natural and chemical fibers, films and sheets of synthetic resins, films and sheets containing animal or plant protein powder or fibers, paper and so forth.

As the lamination process, in case of using polyurethane resins there are one, in which the base material is impregnated with a sponge-containing resin solution, a wet process, in which the solution noted above is coated on the base material, and a dry process, in which a sponge-containing film or sheet is produced from the solution noted above or the like and is laminated either directly or via adhesive on the base material.

In the case of using polyvinyl chloride resins, a thermally fused sponge-containing resin is extrusion or calender molded to obtain a film or a sheet, which is laminated on a base material with an adhesive coated thereon and dried in advance.

Powdery or fibrous sponge may be incorporated in the adhesive layer to improve the moisture permeability.

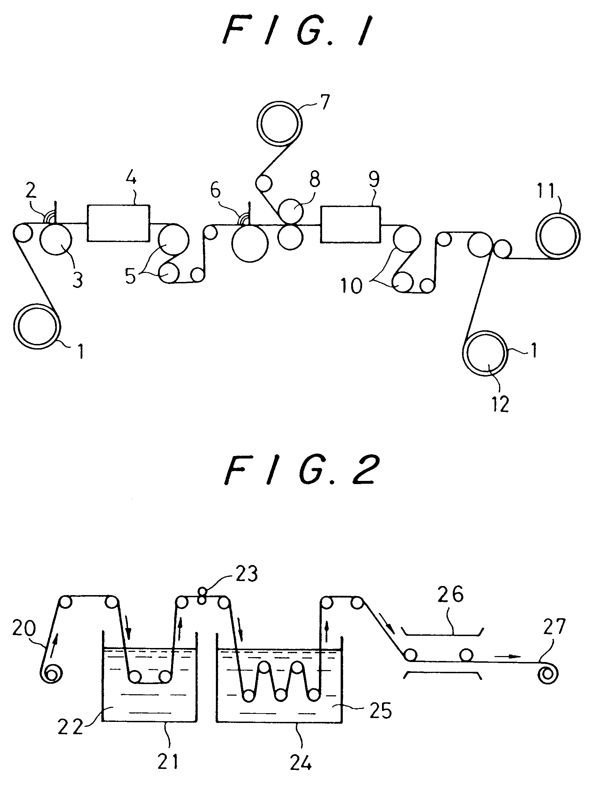

Figs. 1 to 3 show specific lamination apparatuses.

Fig. 1 shows a dry coating apparatus. Dry coating solution 2, which is a sponge-containing resin solution, is coated on a carrier paper sheet 1 to a desired thickness by a knife roller 3, then dried in a drier 4 and then cooled down by cooling rollers 5 to obtain a sponge-containing film or sheet. Then, adhesive 6 is coated on the surface of the film or sheet, and the film or sheet is then laminated on a separately supplied base material 7 (which may be capable or incapable of impregnation) by nip rollers 8, followed by drying in a drier 9 and cooling by cooling rollers 10. In this way, a laminate 11 is obtained. The carrier paper sheet 1 is separated and taken up on a roll 12.

Fig. 2 shows an impregnation coating apparatus. An impregnatable base material 20 is introduced into an impregnating trough 21 and impregnated by an impregnating coating solution 22, i.e., a sponge-containing solution. Then extra solution was squeezed out by mangles 23, and then introduced into a solidifying/washing trough 24 to be solidified and washed by water 25, followed by drying through a drier 26 to obtain a laminate 27.

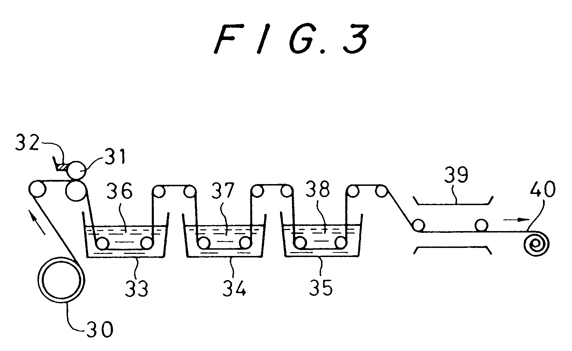

Fig. 3 shows a wet coating apparatus similar to the apparatus shown in Fig. 2. A base material 30 is coated with a wet coating solution 32, i.e., a sponge-containing solution, by a coating roller 31, and then introduced into successive solidifying/washing troughs 33 to 35 to be solidified and washed by water 36 to 38, followed by a drier 39 to obtain a laminate 40.

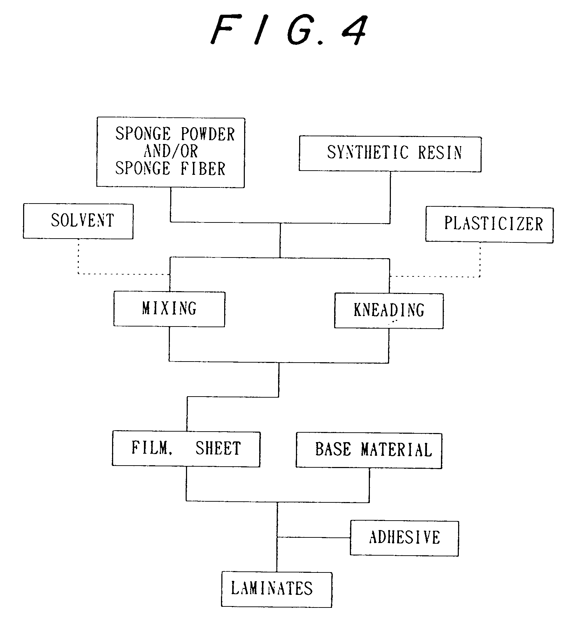

Fig. 4 collectively show the processes of manufacturing the film or sheet and laminate according to the invention.

Conceivable products obtainable from the sponge-containing composition or film or the like prepared from the composition are as follows.

Sponge-containing synthetic resins may be used to produce films, sheets, various plastic moldings and other products.

Rubber may be used to produce such products as shoes (i. e., shoe soles and rubber shoes), OA apparatuses (ruber rollers, keyboards, etc.), industrial apparatuses (i.e., rubbers for printing machines), grips (i.e., golf clubs, bats, tennis rackets, bicycles, etc.), balls (i.e., soft tennis balls, soft balls. etc.), vehicles (i.e., tires, window frame rubber, wiper rubber, vibration-proof rubber, sheets, vehicle interior materials, bicycle brake pads, etc.), office supplies (outer cylinders of writing tools, anti-slip members of fountain pens, desk mats, ink pads, etc.), rubber sheets (vibration-proof members) and rubber-lined cloth (i.e., raincoats).

Laminates of films and sheets may be used to produce such products as synthetic or artificial leather, coats, window breakers, slacks, skirts, jackets, gloves, other clothing, vehicle interior materials, handle leather, handrail covers, furniture (chairs, sofa sheet materials, wall paper, corner cushioning materials, bag materials, shoe materials, shoe inner materials, sanitary products and diaper covers.

(5) Paints and ink

(5-1) Paints

The paints according to the invention contain sponge-containing compositions and solvents and may incorporate various additives and hardening agents, if necessary. As the resin for the paint according to the invention may be used vinyl chloride resins, epoxy resins, acrylic acid resins, urethane resins phenol resins and vinyl acetate resins. Preferred are double liquid type urethane resins, more specifically acrylic acid type urethane resins, polyester type urethane resins, polyether type urethane resins, polycaprolactam type urethane resins, polycarbonate type urethane resins, silion-denatured urethane resins, amino acid-denatured urethane resins and polyamide-denatured urethane resins.

As the solvent for the paint according to the invention may be used those of hydrocarbon type (e.g., toluene and xylene), alcohol type (e.g., ethanol and cyclohexanol), ester type (e.g., ethyl acetate and butyl acetate), ketone type (e.g., acetone and cyclohexanone) and ether type (e.g., ethyleneglycol monoethylether) either alone or in combinations.

Examples of the additive are anti-settling agents, dispersing agents, ultra-violet absorbers and ultra-violet scatterers. As the hardening agent, there polyisocyante and so forth.

By incorporating powdery and/or fibrous sponge in the paint as according to the invention, it is possible to provide such features as good touch, moisture absorption, anti-bacteria property, matting, suppression of fingerprint formation and ultra-violet cutting.

The main applications of the paint according to the invention are to audio products (e.g., TV, audio, video and radio cassettes), OA apparatuses, vehicle interior and exterior parts, electronic and electric products, cosmetic containers, lacquerware, tableware, general goods and every other field.

(5-2) Ink

The ink composition varies with the purpose and method of use. Generally, however, its main constituents are pigments, resins, solvents and additives.

The pigments includes coloring pigments and extender pigments, and resins are selected in dependence on the adhesion, dispersion of pigment, fitness to printing and so forth. The solvent serves to dissolve the resin, and it is selected from the considerations of the fitness to printing, drying property and so forth. Inks of ultra-violet hardening type (i.e., solvent-free inks) are finding increasing applications from the considerations of the toxity and polution aspects. As the additive, there are de-foaming agents, leveling agnts, printing fitness imparting agents and viscosity adjusters. The leveling agent may be those of silicone type, fluorine type and acrylic acid type and also surface active agents, and it is added in an amount of 0.5 to 1.0 % by weight.

The resin used varies depending on the use and kind of print. For example, resins of acrylic acid type, vinyl chloride type, vinyl acetate type, polyester type, cellulose type, epoxy type, meramine type, various polyol and alkid types and cyclized rubber type, as well as various elastomers and acrylate resins, may be used alone or in combinations.

General solvents either alone or in combinations for inks are those of alcohol type (e.g., ethanol and isopropyl alcohol), those of ether type (e.g., n-butylether and dioxane), those of ketone type (e.g., MIBK (methylisobutylketone), diacetone alcohol, dichlohewxane and isohorone), polyhydric alcohol derivatives (e.g., ethyl cellosolve, butyl cellosolve, cellosolve acetate, butyl cellosolve acetate and butylcarbuvitol), those of aromatic hydrocarbon type (e.g., xylol and sorbent naphsa), those of aliphatic hydrocarrbon type (e.g., mineral spirit), and those of halogen type (e.g., trichloroethylene and O-dichlorobenzene).

The following different inks are used depending on the print matter.

For inks used for paper, the resin may be petroleum resins, rosin derivatives, cyclized rubber, chlorinated rubber, chlorinated PP (polypropyrene), polybutadiene, polybutene, EVA (ethylene-viny aceate copolymer), nitrified cotton, alkid, etc., and the solvent may be turpentine, turpentine oil, xylol, etc.

For inks used for hard vinyl chloride (used for sign-boards or the like), the resin may be vinyl choride/vinyl acetate copolymer resin, etc., and the solvent may be xylo, butyl cellosolve, cellosolve acetate, etc.

For inks used for soft vinyl chloride (used for synthetic leather products such as skiing and golfing gloves, wall paper, handbooks, shoes, toys, etc.), the resin may be vinyl chloride/vinyl acerate copolymer, etc., and the solvent may be xylol and butyl cellosolve.

For inks used for acryle (used for interior decoration, outdoor molded signboards, etc.), the resin may be acrylic acid resins, etc., and the solvent may be butyl cellosolve, diacetone alcohol, etc.

For inks used for polyester (used for steckers, electric products and front panels), the resin may be linear saturated polyester, polyuethane resins, etc., and the solvent may be cellosolve acetate and xylol.

For inks used for polycarbonate (used for electric products and front panels), the resin may be vinyl chloride/vinyl acetate copolymer, polyester resins, etc., and the solvent is butyl cellosolve, xylol, etc.

For inks used for polystyrene (used for tableware), the resin may be acrylic acid resins, etc., and the solvent may be butyl cellosolve, n-butanol, etc.

For inks for polyolefin, the resin may be epoxy resins, polyamide resins, chlorinated PP, petroleum resins, EVA and polyurethane resins, etc., and the solvent may be xylol, n-butanol, cellosolve acetate, butyl acetate, etc.

For inks for synthetic leather, the resin may be urethane resins, double liquid type polyurethane resins, etc., and the solvent may be DMF, dimethylformamide, 1,4-butanediol, ethylene glycol, etc.

For inks used for metals, glass and porcelains, the resin may be alkid resins, polyurethane resins, epoxy resins, etc., and the solvent may be cellosolve acetate, xylol, butyl cellosolve, n-butanol, diacetoone alcohol, etc.

(5-3) Powdery and fibrous sponge incorporated in paint and ink

The amount of the powdery and/or fibrous sponge incorpporated in the paint according to the invention is 1 to 90 % by weight, preferably 1 to 60 % by weight. The grain size of the powdery sponge is 100 μm or below, preferably 30 μm or below. The length of the fibrous sponge is 1 cm or below, preferable 5 mm or below. If the amount is excessive, the adhesion to the base material is reduced, and also the mechanical strength of the cover film is reduced. If the grain size or fiber length is excessive, a feel of an alien substance is sensed by touching.

By incorporating the sponge component as according to the invention, it is possible to impart the ink with good sense of touch, moisture absorption property, anti-bacteria property, property of suppressing the fingerprint attachment and property of cutting ultra-violet radiation.

(5-4) Process of coating or printing

The paint according to the invention may be coated in the same way as with the usual point, such as by brushing, immersion, spraying and other ways.

The ink according to the invention may be used in printing in the same way as the usual ink, specifically in the following ways.

The screen printing may be used for packing paper, paper boxes, envelopes, posters, stationeries, plastics, steckers, soft vinyl toys, name plates, T shorts, labels, etc.

The gravure printing may be used for packing paper, paper boxes, envelopes, cards, posters, labels, packing films (PVC (polyvinyl choride), processed PP), construction materials (e.g., wall paper, desks, etc.), etc.

The thermal transfer printing may be used for clothing such as casual wear, sport wear and rain coats, bedding, umbrellas, bags, leather products, lacquerworks, etc.

(6) Cosmetics

The cosmetics according to the invention may be utilized particularly as oily cosmetics.

As usual oily cosmetics, roug, mascara, eye liner, manicure, foundation, sweat suppression stick are well known. The inventor found that incorporation of sponge in the form of powder or fibers of combination thereof into the above oily make-up cosmetics permits imparting the cosmetics with such effects as natural luster, a sense of being damp, an anti-charging effect and an ultra-violet cutting effect. As an example, fibers of rayon, nylon, polyester, cotton, wool, linen, silk, etc. are uniformly dispersed in the base coat which is coated as a ground of manicure to reinforce the nails. By incorporating sponge fibers with lengths of 0.1 to 5 mm, preferably 0.5 to 3 mm in such base coat it is possible to impart the base coat with a sense of being damp, anti-bacteria property and an ultra-violet cutting effect.

Further, it is well known short sponge fibers incorporated in packs for the purpose of removing skin wastes have an effect of increasing the effect of the packs and mechanical strength of generated films. By incorporating sponge fibers with lengths of 0.1 to 5 mm, preferably 0.5 to 5 mm, it is possible to impart a sense of being damp, anti-bacteria effect and ultra-violet cutting effect.

Further, it is possible to impart ductility by incorporating powdery sponge with the average grain size of 15 $\mu$m or below and the maximum grain size of 50 $\mu$m or below.

It is conceivable to use other animal protein in the form of powder and/or fibers to impart a sense dampness like that obtainable with sponge. However, sponge is satisfactory in thermal character even in its non-treated state, and it can be utilized at ease without need of introducing cross-links by means of tanning even in direct application to the skin.

The oil component used for the cosmetics according to he invention is like those broadly used for general oily cosmetics. Its examples are carnauba was, beeswax, ceresine, fluid paraffin, , stearic acid, olive oil, vaseline, alcohol, lanolin, isopropyl myristate, cetanol, and triglycerin 2-ewthylhexanoate.

The sponge component is incorporated by 1 to 90 % by weight, preferably 1 to 60 % by weight. As the other component of the cosmetic, pigment or powder or fibers other than the sponge component, like the case of the usual oily cosmetic, may be incorporated by 0.1 to 40 % by weight with respect to the overall oily cosmetics.

The oily cosmetics according to the invention may incorporate moisture retainer, various chemical components, active agent, perfume, anti-corrosion agent, etc. in addition to the above essential components.

(7) Fibers

Base material fibers used for fibers, which are obtainable by spinning paste compositions obtained by kneading the sponge component according to the invention together with at least one spinning material compound in the group consisting of synthetic fibers, semi-synthetic fibers and regenerated fibers, or fibers with the sponge component attached thereto, are as follows.

Synthetic fibers are those of nylon, polyester, polyacryonitrile, polyvinyl choride, polyviniden chloride, polyvinyl alcohol, polyethylene, polypropyrene and polyurethane. Semi-synthetic resins are those of acetate,, diacetate and triacetate. Regenerated fibers are rayon fibers.

The spinning may be done in the usual way, such as fusion spinning, dry spinning, dry wet spinning and wet spinning.

The sponge component is added by 1 to 60 % by weight, preferably 1 to 30 % by weight. The grain size of the powdery sponge is 20 $\mu$m or below, preferably 10 $\mu$m or below.

When the amount added or the grain size is increased, breakage may occur, making the spinning difficult and also reducing the mechanical strength of the spun fibers.

By incorporating the sponge component in fibers according to the invention, it is possible to impart good touch, moisture absorption property, anti-bacteria property and ultra-violet cutting effect. Further, similar properties can be provided to woven and non-woven cloth made of these fibers.

Further, the sponge component may be used not only by kneading it with the base material fibers but also by attaching powdery or fibrous sponge to the surface of spun fibers to cover the fiber surface. By so doing, the same effects as in the case of the kneading can be obtained.

(8) Fiber-finishing materials

Of the sponge-containing compositions according to the invention synthetic resins used as fiber-finishing materials are, for instance, polyurethane resins, silicon resins, polyester resins, fluorine resins, etc.

The fiber finishing processes, fibers used for the processes and characteristics of the fibers are as follows.

For the super-soft process, mainly silicon resins and polyurethane resins are used.

As the silicon resin, amino-denatured silicon is mainly used in view of super-soft sense and draping property. The polyurethane resins impart a sense of volume, a sense of repulsion and a sense of dryness in addition to the flexibility, and it has great effects when it is used in combination of silicon resins.

In the super-soft process, there are cases of incorporating small amounts of usual flexibility promoters to impart the water absorption property and hard finishing agents to impart a sense of volume and a sense of dryness.

In a peach skin process, fiber material is emery raised, followed by padding or coating of a silicon resin (to impart a soft touch) and a polyurethane resin (to impart a sense of volume and a sense of repulsion).

As other processes, fluorine resin is used for water- and oil-repelling processes, polyurethane resin and polyester resin are used in combination for touff pil process, and for a clogging process polyurethane resin (of water absorption type for bedding material) or combination of silicon resin and fluorine type water-repelling agent (for outer) is used.

As the urethane resin used for finishing in the fiber finishing process, those of single liquid type (thermosetting) have high surface strength and used for the outermost layer, while those of the double liquid type (thermosetting) have close contact property and can find extensive applications as coupling layers with respect to fibers.

With fibers, improvements of the performance and functions are important. For example, high durability is required for furniture and vehicles, and moisture permeable function of imparting moisture permeability while maintaining water-proof property is required for sports garments.

The former requirement is met by the pil process or the like. To meet the latter requirement, use has been made of a moisture absorbing/emitting mechanism which is provided by introducing a hydrophilic component into urethane soft segments. Alternatively, a special component having a three-dimensional helix structure is introduced into soft segments to make use of a permeation mechanism of the gaps in the three-dimensional structure. These measures, however, are expensive and can not sufficiently fulfill the function of moisture permeation.

In contrast, the incorporation of the sponge component into the fiber-finishing material has the following effects.

By merely incorporating the sponge component in various fiber-finishing materials, it is possible to impart a moisture absorbing/emitting mechanism, a sense of touch to natural materials, anti-bacteria property and an ultra-violet cutting property.

The average grain size of the powdery sponge added to the fiber-finishing material is 60 μm or below, preferably 30 μm or below. If it exceeds 60 μm, a sense of roughness is produced. The fiber length of the fibrous sponge is suitably 5 mm or below. If it exceeds 5 mm, a sense of roughness is produced. The amount of the sponge component used is suitably 1 to 60 % by weight. If it is excessive, the adhesion is reduced.

The sponge-containing compositions, films and like products using such compositions and sponge-containing products according to the invention do not only provide for good touch but also are satisfactory in the moisture absorption and emission properties and also in the thermal properties. Besides, they are satisfactory in the anti-bacteria property, safety and ultra-violet cutting property.

For example, resin moldings and other compositions and products as noted above have the above satisfactory properties. Further, interior materials and construction materials have a sense of good touch and satisfactory moisture absorption and emission and anti-bacteria properties. Garments, diaper covers, sanitary goods, bathing sponge, cosmetic puffs, poultices and emergency adhesive plasters are soft to the skin and have a sense of good touch, anti-bacteria property and so forth. Freshness retaining bags, paper towels and food additives have satisfactory anti-bacteria property. Medical goods (e.g., diaper sheets, wooden pads, and disposable surgical operation garments and gloves) are not only satisfactory in the anti-bacteria property and the sense of touch, but also are highly safe. Further, fishing nets and vinyl house films have satisfactory anti-bacteria property.

In the accompanying drawings:-

Fig. 1 is a schematic view showing a dry coating apparatus used for forming a laminate according to the invention;

Fig. 2 is a schematic view showing a dip coating apparatus for forming a laminate according to the invention;

Fig. 3 is a schematic view showing a wet coating apparatus for forming a laminate according to the invention;

Fig. 4 is a view outlining the processes of producing films, sheets and laminated according to the invention;

Fig. 5 is a schematic top view showing a film applicator used when producing Experiment example 1 according to the invention; and

Fig. 6 is a graph showing a the light transmission factor (i.e., ultra-violet cutting property) obtained with Experiment example and also with Comparative experiment example 1.

How, specific experiment examples according to the invention and comparative experiment examples will be described.

In the first place, the best mode of bleaching, if necessary, will be described from the pulverizing state.

(I) Pulverizing stage

Cutting chips of sponge produced in sponge processing were used as the material. The sponge chips were crushed at a rate of 10 kg of process amount per hour using a vertical crusher ("VM-32" manufactured by Orient Co., Ltd., with screen having a round hole diameter of 2 mm). The resultant crushed sponge was dried in a drier at 120 °C for 2 hours, and was then pulverized for 5 minutes using a vibration mill ("TI-200--50" manufactured by Heiko Seisakusho, Ltd.).

The pulverized sponge was then sheaved for one hour using a sound wave sheave ("SW-20" manufactured by Tsutsui Rikagaku Kikai Co., Ltd.) to extract only particles having passed through sheave screen with a mesh size of 32 μm. The other particles were pulverized again with the above vibration mill ad then sheaved likewise. The operation was repeatedly carried out three times, and the average grain size of the extracted powdery sponge, measured by using a laser refraction grain size analyzer ("SK LASer RO7000S" manufactured by Seishin Kigyo Co., Ltd., dispersoid: ethanol, dispersing conditions: U.S. wave, 60 sec.), was found to be 8 μm.

### (II) Bleaching stage

The pulverized sponge obtained in the above pulverizing state (I) was then bleached.

In the first place, into a 100-ml beaker were put 1.2 g of a chlorine type bleacher ("Niilite L-20" manufactured by Nissei Kasei), 0.1 g of non-ionic active agent ("Sordine SK-F", manufactured by Nissei Kasei Co., Ltd.) and 25 ml of ion-exchanged water. The system was stirred until it was homogenious. During the stirring, the pH of the solution was adjusted to 2.5 by adding oxalic acid.

In the solution thus prepared was dipped 1 g of the above pulverized sponge, which was then left at room temperature for 24 hours for oxidization bleaching. Then, the powder was water washed in the order of filtering, washing and filtering to be ready for chlorine removal.

A chlorine removal solution was prepared by charging 7.5 g of a 35 % aqueous hydrogen peroxide solution, 1 g of a hydrogen peroxide stabilizer ("M-920" manufactured by Nissei Kasei Co., Ltd., 1 g of a non-ionic active agent ("Soldine SK-F" manufactured by Nissei Kasei and 100 ml of ion-exchanged water into a 100-ml beaker and stirring the system until the system was made homogenious while adjusting the pH of the solution to 10 to 11 by adding sodium hydroxide.

In this solution, 1 g of the oxidization bleached powdery sponge was dipped, followed by leaving at room temperature for 24 hours for chlorine removal. Then, the powder was water washed in the order of filtering, water washing and filtering to be ready for reduction bleaching.

A reduction bleaching solution was prepared by charging 1 g of a reduction bleacher ("Niilite KA-NF" manufactured by Nissei Kagaku CO., Ltd.) and 25 ml of ion-exchanged water into a 100-ml beaker and stirring the system until the system became homogenious while adjusting the pH of the solution by adding oxalic acid.

In this solution, the powdery sponge after the chlorine removal was dipped to be left at room temperature for 24 hours for reduction bleaching. Then, the powder was water washed in the order of filtering, water washing and filtering. The powdery sponge thus obtained was held dipped in an acetone solution for 10 minutes. Then, the powder was dried in a state held between filter sheets and then mashed in a mortar using a wooden pestle, thus obtaining white powdery sponge. The average grain size of the powdery sponge was measured and found to be 50 μm.

The whiteness degree W of the powdery sponge was calculated by substituting values of L = 91.13, a = -0.78 and b = 4.24 measured by using a color difference meter ("CR-20" manufactured by Minorta Camera Co., Ltd.) and was found to be W = 90.1.

Now, the best experimental example is shown, which permits providing a sponge-containing composition and a sponge-containing product containing such composition for improving the appearance and such functions as moisture absorption and emission properties of various products without carrying out any bleaching treatment.

### Experiment example 1

### Process of preparing powder and fibers of sponge

As the material was used cutting chips of sponge produced when the sponge was processed. The material sponge chips was crushed using a grind crushing mill until the fiber length become 1 cm or below. As the fibrous sponge according to the invention, this fibrous crushed sponge was used. The crushed sponge was pulverized using a ball mill until the average grain size became 30 μm or below. The pulverized sponge was further finely pulverized using an air stream mill to obtain powder with an average grain size of 5 μm.

The sponge fibers and sponge powder thus obtained were used for the following experiment examples.

### Production of film

A sponge-containing film (sponge film) was produced by using as the film material "Rezamine ME-3812LP", a single liquid type Polyester type polyurethane resin solution manufactured by Dainihon Seika Kogyo Co., Ltd. As a reference sample, a film not containing powdery sponge (blank film) was produced. The process of production was as follows.

(I) 100 g of "Rezamine ME-38121LP" charged into a 200-ml beaker. This substance had 30 % by weight of volatile component.

(II) To this substance was added 60 g of a solvent solution composed of DMF (dimethylformamide) and MEK (methylethylketone in a ratio of 1 : 1.

(III) To this blend solution was added the sponge powder in such weight amounts as to obtain the following predetermined weight concentrations (a), (b) and (c) specifically with respect to the non-volatile component of"Rezamine ME-3812LP".

(a) 20 wt %-6 g, (b) 30 wt %-9 g, (c) 50 wt %-15 g

(IV) Adter adding the powder, the system was stirred using a medical spatula until the system became homogenious. Then, the system was sealed in a wrap and left at room temperature for 24 hours for defrothing.

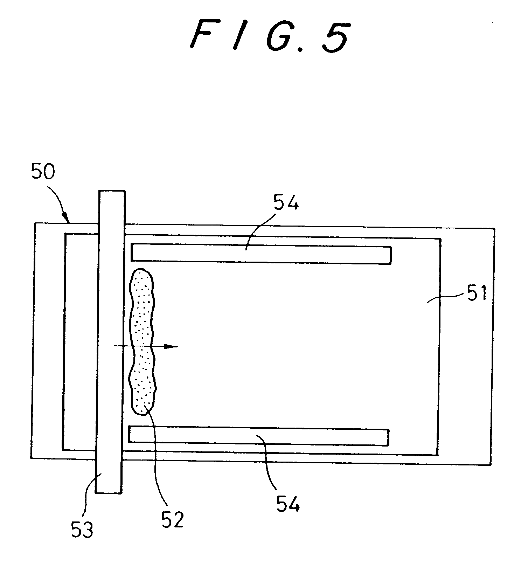

(V) Then, the system was stirred slowly again lest bubbles should be introduced. Then an adequate amount of this solution 52 was dropped onto a separable paper sheet 51 set on a film applicator 50, as shown in Fig. 5.

(VI) A film is formed by moving a coating bar at a constant speed to the right in the Figure. To obtain a film pressure of 20 μm a tape 54 is applied to a portion of the separable paper sheet 51 in the vicinity of the upper and lower edges.

(VII) The applicator 50 is left in a drying oven set at 80 °C for about 10 minutes, thus causing gassification of the solvent and obtaining a film from the resin solution.

(VIII) After the gassification, the film is separated from the separable paper sheet, thus obtaining the desired film.

Comparative experiment example 1

A film without powdery sponge was produced in the manner as in Experiment example 1.

Moisture absorption and emission test

As the test samples the sponge film (b) with the sponge component of 3 % among the sponge films (a) to (c) produced in Experiment example 1 and a blank film produced in Comparative experiment example 1 were used.

Method of test

(I) A film of the same thickness is selected by measuring the thickness.

(II) The film is cut to 12 cm x 12 cm.

(III) The cut film is set on an aluminum sheet of 12 cm x 12 cm, and a vinyl tape is applied along the edges such that the measurement surface is 10 cm x 10 cm.

(IV) The sample is put in a constant temperature, constant relative humidity trough at a temperature of 23 °C and at a relative humidity (RH) of 30 %.

(V) The sample is left for more than 12 hours in this state. (VI) The sample is then taken out of the constant temperature, constant relative humidity trough, and its weight is measured and recorded.

(VII) Then, the sample is put in each of two constant temperature, constant relztive humidity troughs (30 °C, 80 % RH.

(VIII) The weight is measured at an interval of one hour up to four hours, and the measured values are recorded. At this time, the difference of the measurement from the measured value obtained in (VI) is the moisture absorption.

(IX) The sample is put in the first constant temperature, constant relative humidity trough again. The weight is then measured at an interval of one hour up to four hours, and the measured values are recorded. The weight difference of the measurement with respect to the value obtained in the measurement after four hours is the moisture emission.

The results of the test are shown in Table 1. It will be seen from Table 1 that the film incorporating powdery sponge according to the invention has high moisture absorption and emission.

In the test the moisture permeability per unit area is measured, but it is possible to measure moisture absorption and emission per unit weight.

EP 0 551 762 A1

Table 1

| Sample name | Moisture absorption (g/m³) | | | | Moisture emission (g/m³) | | | |
|---|---|---|---|---|---|---|---|---|
| | 1hr | 2hr | 3hr | 4hr | 1hr | 2hr | 3hr | 4hr |
| Exp. example 1(b) | 3.9 | 5.7 | 6.7 | 7.9 | 2.8 | 4.0 | 4.8 | 5.4 |
| Com. example 1 | 2.8 | 4.4 | 5.3 | 6.3 | 2.0 | 3.2 | 3.8 | 4.5 |

Moisture permeability test

As the test samples the sponge film (a) with the sponge content of 2 % by weight among the sponge films (a) to (c) produced in Experiment example 1 and a blank film produced in Comparative experiment example 1 were produced.

The method of test was conformed to JIS L10099.

The results of the test are shown in Table 2. It will be seen from Table 2 that the film incorporating powdery sponge according to the invention has high moisture permeability.

Table 2

| Sample name | Moisture permeability (mg/m²/24 hr.) |
|---|---|
| Exp. example 1(a) | 1802 |
| Com. example 1 | 1402 |

Anti-bacteria power test

As the test samples, the sponge film (c) with the sponge content of 5 % by weight among the sponge films (a) to (c) produced in Experiment example 1 and a blank film produced in Comparative experiment example 1 were used. The method of test conformed to AATCC Method 100.

More specifically, liquid containing colon bacillus was dropped onto a film cut to 5 cm x 5 cm. The film was then preserved to measure the number of bacilli at intervals. The liquid containing bacilli was prepared by culturing for 18 to 24 hours and then adjusting the number of bacilli to 105 to 106 per ml by using anti-bacteria physiological brine.

The result of test is shown in Table 3. It will be seen from Tale 3 that after three hours the number of bacilli with the sponge film in this experiment example is substantially zero, thus indicating the anti-bacteria action of the sponge film.

Table 3

| Sample name | Preservation time (residual bacteria number) | | | |
|---|---|---|---|---|
| | 0 hr. | 1 hr. | 3 hr. | 6 hr. |
| Exp. example 1(c) | 6.2 x $10^5$ | 2.5 x $10^4$ | less than 10 | less than 10 |
| Com. example 1 | 6.2 x $10^5$ | 3.8 x $10^5$ | 2.2 x $10^5$ | 2.0 x $10^5$ |

Ultra-violet permeation test

As the test sample the sponge film (b) with the sponge content of 30 % by weight among the sponge films (a) to (c) produced in Experiment example 1 and a blank film produced in Comparative experiment example were used.

As the method of test, the permeation was measured using an integration ball of "Shimatsu Spectrometer UV-240" manufactured by Shimmatsu Seisakusho.

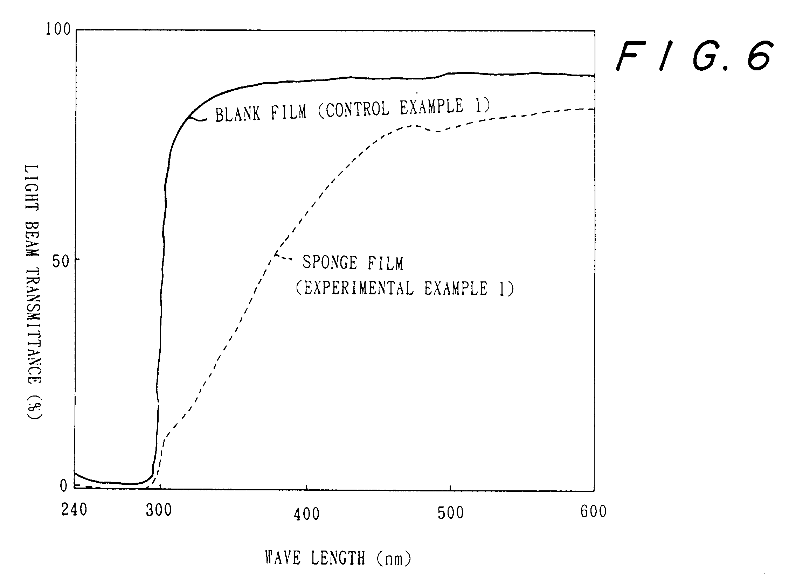

The result of test is shown in Fig. 6.

Generally, of the ultra-violet rays (180 to 400 nm) those of 180 to 290 nm do not reach the earth. Those of 290 to 320 nm cause red swell, drought, blister, etc. of the skin. Those of 320 to 400 nm cause black burn and spots and wrincles and promote aging. Thus, it is effective to reduce wavelengths of 400 nm and below.

It will be seen from Fig. 6 that the sponge film according to the invention cuts ultra-violet rays of 290 to 400 nm.

Experiment example 2

20 % by weight of dry sponge powder was added to 80 % by weight of a soft vinyl chloride resin (manufactured by Shinetsu Polymer Co., Ltd., plasticizer content: 30 % by weight, mean polymerization degree: 3,000). The mixture was kneaded while being heated at 160 °C, and formed into a sheet and then into pellets. The pellets thus obtained were used to form a sheet with a thickness of 400 µm using a press molder.

Experiment example 3 (dry synthetic leather)

As dry coating liquid, a solution obtained by slightly adding auxiliary additive to a dry polyUrethane resin solution incorporating 6 % by weight as solid content of dry sponge powder was coated on a separable paper sheet using the dry coating apparatus shown in Fig. 1, followed by drying at 130 °C, thus obtaining a polyurethane film containing powdery sponge with a thickness of 20 µm. This film was bonded a base material, i.e., non-woven polyester cloth of one denier and with a weight of 70 g/cm2, by using a polyurethane type adhesive incorporating 5 % by weight as solid component of dry sponge powder and then dried, thus obtaining a sheet having a polyurethane resin layer containing sponge powder with a thickness of 40 µm for dry synthetic leather.

Experiment example 4 (wet synthetic leather)

As wet coating liquid a solution obtained by slightly adding a filler to a wet polyurethane resin solution incorporating 30 % by weight as solid content of dry sponge powder was coated on a base material, i.e., a non-woven cloth of blend fibers containing 50 % by weight of nylon and 50 % by weight of Tetoron using the wet coating apparatus shown in Fig. 3 and then dried at 140 °C, thus producing a sheet having an ester type polyurethane resin layer 0.7 mm thick and containing sponge powder for wet synthetic leather.

Experiment example 5 (artificial leather)

As impregnation coating liquid, a solution obtained by adding three parts of a filler to an ester type polyester resin solution incorporating 25 % by weight in solid content of dry sponge powder (with a resin content of 15 % by weight) was prepared, and a base material, i.e., a non-woven polyester cloth of 2 denier and with a weight of 70 g/m2, was impregnated with 30 g/m2 in dry weight of the above solution using the impregnation coating apparatus shown in Fig. 2 and dried at 130 °C.

Then, as wet coating liquid a solution obtained by adding the same filler as noted above and a foaming agent to an ester type polyurethane resin solution incorporating 30 % by weight as solid content of dry sponge powder (with a resin content of 15 % by weight) was prepared. The solution thus prepared was coated on the impregnated base material obtained as above using the wet coating apparatus shown in Fig. 3 and then dried at 130 °C, thus obtaining a sheet a porous polyurethane resin layer having a thickness of 0.4 mm for artificial leather.

Comparative experiment examples 2 to 5

Sheets or laminates were produced in the same manner as in Experiment examples 2 to 5 except that no sponge powder was used.

Moisture absorption and emission test

The films, sheets and laminates obtained in the above ways were tested for their moisture absorption and emission performance.

The method of test was the same as that of the moisture absorption and emission test in Experiment example 1.

The measurement was made at an interval of 20 minutes (one-third hour) up to one hour and then at an interval of one hour up to 24 hours.

The result is shown in Table 4. It will be seen from Table 4 that with the sample in this experiment example high moisture absorption and emission can be obtained.

## Table 4

|  | Moisture absorption ($g/m_2$) | | | Moisture emission ($g/m_2$) | | |
|---|---|---|---|---|---|---|
|  | 1/3Hr | 1Hr | 24Hr | 1/3Hr | 1Hr | 24Hr |
| Exp. ex. 2 | 1.3 | 2.4 | 13.6 | 1.0 | 2.3 | 4.0 |
| Com. ex. 2 | 0.2 | 0.5 | 2.0 | 0.2 | 0.6 | 0.9 |
| Exp. ex. 3 | 2.6 | 3.5 | 5.1 | 1.8 | 3.1 | 4.4 |
| Com. ex. 3 | 1.2 | 1.5 | 2.4 | 0.7 | 1.2 | 2.0 |
| Exp. ex. 4 | 13.0 | 15.4 | 22.3 | 12.0 | 15.0 | 22.0 |
| Com. ex. 4 | 8.1 | 9.8 | 12.1 | 7.0 | 9.0 | 11.4 |
| Exp. ex. 5 | 9.6 | 10.3 | 13.0 | 8.0 | 10.2 | 12.9 |
| Com. ex. 5 | 2.9 | 4.0 | 5.4 | 2.7 | 4.0 | 5.3 |

Sensual test

The samples obtained in Experiment examples 2 to 5 and Comparative experiment examples 2 to 5 were touch tested by ten persons, and their performance was judged as the average of evaluation values obtained in the following.

In the test, the sample surface was touched with the hand for comparison.

Point 5: The touch is very good. Point 4: The touch is good. Point 3: The touch is usual. Point 2: The touch is bad. Point 1: The touch is very bad.

The result is shown in Table 5. It will be seen from Table 5 that the touch is better with the samples obtained in the experiment examples and that it is usual or worse .

Table 5

| Exp. example 2 | 4.3 |
|---|---|
| Com. example 2 | 2.7 |
| Exp. example 3 | 4.4 |
| Com. example 3 | 3.0 |
| Exp. example 4 | 4.3 |
| Com. example 4 | 2.9 |
| Exp. example 5 | 4.4 |
| Com. example 5 | 3.0 |

Experiment example 6

30 % by weight of th dry fibrous sponge obtained in Experiment example 1 and 70 % by weight of L-LDPE (linear chain low density polyethylene, manufactured as "Moretec 1018T" by Idemitsu Sekiyo Kagaku Co., Ltd.) were mixed. The mixture was heated, kneaded and extruded into pellets. The pellets were then injection molded to obtain a sheet-like injection molding product (having a horizontal dimension of 80 mm, a vertical dimension of 80 mm and a thickness of 3 mm).

Test on molding product

The molding was left in an atmosphere at 30 °C and under 95 % RH for 24 hours, and then white powder generation on the surface of the molding was checked for, and no generation of white powder could be found.

Experiment example 7

70 % by weight of polyuirethane elastomer ("Elastoran C80A10" manufactured by Takeda Badish Urethane Industries) having been fused to 180 °C with a Bumbury's mizer and 30 % by weight of dry fibrous sponge prepared in Experiment example were mixed and kneaded together. The paste block thus obtained was then formed into a sheet using paste rollers. From this sheet, granules were produced and then processed in the manner as in Experiment example 6 to obtain an injection molding product of the same size.

The molding was then checked by the same method of test as in Experiment example 6 to find no white powder generation.

Experiment example 8

30 % by weight of the dry fibrous sponge prepared in Experiment example 1 and 70 % by wight of EPDM (ethylenepropylene copolymer "KELTAN.IS-50A" manufactured by Idemitsu Co., Ltd.) were mixed and kneaded together. To 100 parts by weight of the mixture was added 1.5 parts by weight of a vulcanizer, and the admixture was kneaded using kneading rollers at a surface temperature of 40 to 60 °C and then immediately press molded to obtain a sheet-like press molding product (having a vertical dimension of 150 mm, a horizontal dimension of 150 mm and a thickness of 1 mm).

This molding was checked by the same method of test as in Experiment example 6 to find no white powder generation.

Experiment example 9

30 % by weight of the dry fibrous sponge prepared in Experiment example 1 and 70 % by weight of a butadiene resin ("RB820" manufactured by Nihon Gosei Gumu Co., Ltd.) were mixed and kneaded together at a temperature of 110 to 130 °C by using a Bumbury's mixer, then formed into a sheet using two rollers and then formed into pellets.

The pellets were then press molded to obtain a sheet like press molding product (with a vertical dimension of 150 mm, a horizontal dimension of 150 mm and a thickness of 1 mm). The molding was checked by the method of test in Experiment example 6 to find no white powder generation.

Experiment example 10

30 % by weight of the dry fibrous sponge prepared in Experiment example 1 and 70 % by weight of a powder slash material, which is composed of 100 parts by weight of a straight vinyl chloride resin with a polymerization degree of 800 to 1,000 and an average grain size of 150 μm, 70 parts by weight of dioctylphthalae as a plasticizer and 30 pars by weight of straight vinyl chloride powder with an average grain size of 10 μm for denaturing were mixed. The mixture was stirred using a (B) mixer at a temperature of 150 to 170 °C. The obtained powder was charged into a die held at a temperature of 230 °C for molding for about 10 seconds. Then, after removing non-fused portion, the system was further molded for 40 seconds, thus obtaining a sheet-like slash molding (with a vertical dimension of 100 mm, a horizontal dimension of 100 mm and a thickness of 2 mm). A check as in Experiment example 6 was made to find no white powder.

Comparative experiment examples 6 to 10

As Comparative experiment examples, injection molding products free from fibrous sponge were obtained respectively in the same manners as in Experiment examples 6 to 10.

Sensual test

The touch test was conducted on samples of Experiment examples 6 and 10 and Comparative experiment examples in the manner as in Experiment examples 2 to 5. The result s shown in Table 6. It will be seen from Table 6 that samples in the experiment examples has a sense of good touch, whereas those in the comparative experiment examples has at most usual touch.

Table 6

| | |
|---|---|
| Exp. example 6 | 4.5 |
| Com. example 6 | 2.6 |
| Exp. example 7 | 4.3 |
| Com. example 7 | 3.0 |
| Exp. example 8 | 4.2 |
| Com. example 8 | 2.9 |
| Exp. example 9 | 4.3 |
| Com. example 9 | 3.0 |
| Exp. example 10 | 4.3 |
| Com. example 10 | 2.9 |

Experiment example 11

A paint, which was obtained by adding 20 % by weight of the powdery sponge prepared in Experiment example 1 to a main material using polyester polyol as resin and polyisocyanate, i.e., HMD1 trimmer, as hardening agent and also adding a blend solution containing toluene, xylene and butyl acetate and such additives as a dispersing agent, an anti-settling agent and an ultra-violet absorber, was blown against a flat surface of ABS resin to form a coating film.

Comparative experiment example 11

A paint free from sponge powder was produced in the same manner as in Experiment example and blown against a plane surface of ABS resin to form a coating film.

Sensual test

Samples in Experiment example 11 and Comparative experiment example 11 were tested with respect to the touch and fingerprint adhesion by 10 persons in a four-point evaluation method. The result is shown in Table 7. It will be seen from the table that the sample of the experiment example has a sense of very good touch and is difficult to attach a fingerprint whereas the sample in the comparative experiment example has a sense of bad touch and is ready to attach fingerprint.

Table 7

|  | Touch | Fingerprint at-tachment |
|---|---|---|
| Exp. example 11 | ◎ | ◎ |
| Com. example 11 | Δ | × |

◎      ○      Δ      ×
← Touch is good    Touch is bad →
Fingerprint is       Fingerprint is
difficulty attached    readily attached

Example 12

An ink was produced by incorporating, in an ink having a composition as shown in Table 8, 20 % by weight with respect to resin of the sponge powder prepared in Experiment example 1. This ink was used for printing on a foamed polystyrene sheet.

Table 8

| Incorporated material | Part by weight |
|---|---|
| Acrylic acid resin | 25 |
| Phthalocyanines blue | 8 |
| Butyl cellosolve | 33 |
| n-butanol | 30 |
| Coating film character improver | 4 |
| | 100 |

Comparative experiment example 12

An ink free from sponge powder was produced in the manner as in Experiment example 12, and it was used for printing no a foamed Polystyrene sheet.

Sensual test

Sensual test on samples in Experiment example 12 and Comparative experiment example 12 was made by 10 persons with respect to the touch and fingerprint attachment in a four-point evaluation method. The result is shown in Fig. 9. It will been from the Figure that the sample in the experiment example is a sense of good touch and is difficult to attach a fingerprint to it, whereas the sample in the comparative experiment example has a sense of bad touch and is ready to attach a fingerprint to it.

Table 9

|  | Touch | Fingerprint attachment |
|---|---|---|
| Exp. example 12 | ◎ | ◎ |
| Com. example 12 | △ | △ |

```
     ◎          O          △          ×
 ← Touch is good        Touch is bad →
   Fingerprint is       Fingerprint is
   difficulty attached  readily attached
```

The invention now will be described in detail in conjunction with oily cosmetics. More specifically, Experiment examples 13 to 15, in which the sponger powder prepared in Experiment example 1 is incorporated, and Comparative experiment examples 13 to 15, in which a conventional affine-to-oil surface active substance is incorporated, will be described.

Experiment example 13,

Comparative experiment example 13 (eye liner)

The sponge powder prepared in Example 1 was incorporated into an eye liner, and the effect of the invention was checked.
The process of production is as follows.
Mixtures as shown in A in Table 10 were thermally fused. To the fused compositions were added components shown in B. The resultant systems were dispersed using three rollers to obtain eye liners in Experiment example 13 and Comparative experiment example 13.

Table 10

| Incorporated material | | Exp. example 13 (part by weight) | Com. example 13 (part by weight) |
|---|---|---|---|
| A | Microcrystalline wax | 10.0 | 10.0 |
| | Carmaiba wax | 1.0 | 1.0 |
| | Dimethyl silicon (1cs) | 61.0 | 61.0 |
| | Isopropyl myrist-ate | 5.0 | 5.0 |
| | Sorbitan monoiso-olate | - | 2.0 |
| B | Titanium oxide | 5.0 | 5.0 |
| | Ultramarine | 15.0 | 15.0 |
| | Powdery sponge prepared in Exp. 1 | 3.0 | - |

Charging test

A sample containing the sponge powder prepared in Experiment example and that without sponge powder were tested for charging as follows.

A negatively charged nylon cloth piece 10 cm in one side was hung from a stand, and a stainless steel microspatula, to which about 0.1 g of each of the samples in the experiment example and comparative experiment example was attached, was brought to a position at a distance of 10 cm from the hanging cloth piece to check the dispersion state of the eye liner.

The eye liner was not dispersed at all with the product in the experimental example, whereas it was extremely dispersed in the product in the comparative experiment example, thus contaminating the nylon cloth.

Experiment example 14,

Comparative experiment example (sweat suppression stick)

The effect of the sponge powder prepared in Experiment example 1 was checked with the sweat suppression stick.

The process of production of the sweat suppression stick was as follows.

The compositions shown in A in Table 11 were thermally fused, then the components shown in B are mixed, and then systems were dispersed with a high speed disperser. These compositions were then each charged into a component die and solidified by cooling, thus obtaining sweat suppression sticks in Experiment example 14 and Comparative experiment example 14.

Table 11

| Incorporated material | | Exp. example 14 (part by weight) | Com. example 14 (part by weight) |
|---|---|---|---|
| A | Solid paraffin | 8.0 | 8.0 |
| | Beeswax | 5.0 | 5.0 |
| | Fluid paraffin | 10.0 | 10.0 |
| | Glycelyl triiooctanate | 63.0 | 63.0 |
| | Glycerylsesquiolate | - | 2.0 |
| B | Zinc oxide | 3.0 | 3.0 |
| | Aluminum chlorohydrate | 2.0 | 2.0 |
| | Cellulose powder | 5.0 | 5.0 |
| | Powdery sponge prepared in Exp. example 1 | 4.0 | - |

Charging test and sensual test

A sample containing the sponge powder prepared in Experiment example 1 and a sample not containing sponge powder were compared in a charging test and a sensual test.

The result of the charging test was as follows.

The sweat suppression sticks in the experiment example and the comparative example were left overnight in a room under a relative humidity of 40 % and at a temperature of 20 °C, and the amount of dust attached to the stick surface was checked.

With the sample in the experiment example, only slight dust was attached to the stick surface. With the sample in the comparative experiment example, a large mount of dust was attached. A major portion of the attached dust was constituted by negatively charged chemical fibers.

The result of the charging test is as follows.

The sweat suppression sticks in the experiment example and comparative experiment example were used by 18 to 22 women for hair-free armpits for their sensual evaluation with respect to the sense of stickiness and sense of dampness by a five-point method. The average value of each item with ten values is as shown in Table 2. It will be seen from Table 1 that the sample in the experiment example has less sense of stickiness and more sense of dampness compared to the sample in the comparative experiment example.

Table 12

| Test item | Evaluation by sensual test | |
|---|---|---|
| | Exp. example 14 | Com. example 14 |
| Sense of stickiness | 1.0 | 3.9 |
| Sense of dampness | 4.0 | 1.8 |

Experiment example 15,

Comparative experiment example 15 (rouge)

The effect of the sponge powder prepared in Experiment example 1 in rouge was checked.

The process of production of the rouge was as follows.

The components in A in Fig. 13 were mixed and thermally fused. Then, the components shown in B were added. The components were then dispersed by using three rollers. The dispersoids were each charged into a molding die and cooled down to be solidified, thus obtaining samples in Experiment example and Comparative experiment example.

## Table 13

| | | Exp. example 15 (part of weight) | Com. example 15 (part by weight) |
|---|---|---|---|
| A | Microcrystal-line wax | 10.0 | 10.0 |
| | Carnauba wax | 2.0 | 2.0 |
| | Candelilla wax | 6.0 | 6.0 |
| | Lanolin | 20.0 | 20.0 |
| | Isopropyl myr-istate | 10.0 | 10.0 |
| | Caster oil | 46.0 | 46.0 |
| | Sorbitan mono-isostearate | – | 2.0 |
| B | Titanium oxide | 2.0 | 2.0 |
| | Red No. 202 | 1.0 | 1.0 |
| | Powdery sponge prepared in Exp. example 1 | 3.0 | – |

Hardness measurement and sensual test

A sample containing the sponge powder prepared in Experiment example and a comparative sample not containing sponge powder were compared with by a hardness measurement and a sensual test.

The hardness of the rouge was measured at 25 °C by using a card tension meter. In the experiment example, the hardness was 200 g, whereas in the comparative experiment example it was 140 g. The experiment example could improve the hardness reduction.

For the comparison by the sensual test, the rouge in the experiment example and that in the comparative experiment example were used by 18 to 22 women, and the sense of roughness after coating was evaluated by a five-point method. The average values each of the ten individual values were shown in Table 14. From the table it will be seen that withe the rouge in the experiment example the sense of roughness is less.

## Table 14

| Test item | Evaluation by sensual test | |
|---|---|---|
| | Exp. example 15 | Com. example 15 |
| Sense of roughness | 2.4 | 4.2 |

With Experiment examples 13 to 15 it is possible to obtain far superior anti-charging effect of oily cosmetics without deteriorating the sense of use and quality. In addition, it is possible to impart natural luster without sense of roughness and also impart a sense of dampness.

The stick cosmetics include not only eye liners, sweat suppression sticks and rouges, but also lipsticks, eyebrow pencils and other stick-like cosmetics.

The invention will now be described in detail in conjunction with fibers. More specifically, Experiment examples 16 to 18, in which the sponge powder produced in Experiment example 1 was kneaded and spun with or attached after spinning to various resin base materials, and comparative experiment examples 16 to 18, which do not use sponge powder, will be described.

Experiment example 16

Dimethyl formamide (DMF) was added to a polyester type urethane resin solution such that the non-volatile component was 20 % by weight. Then, sponge powder prepared in Experiment example 1 was added by 20 % by weight with respect to the non-volatile component, thus obtaining a spinning solution. After sufficient kneading and defoaming, the urethane fibers containing powdery sponge were spun by a wet spinning process, in which the spinning solution was extruded from a spinning die into a water agglomerating bath by a gear pump.

Experiment example 17

30 % by weight of the sponge powder prepared in Experiment example 1 was blended with pellet-like polypropyrene and also with polypropyrene using a blender. Each blend sample was fusion extruded from a 30-mm uni-axial extruder, then cooled down and then cut to obtain pellets containing powdery sponge. The pellets containing powder sponge were used as master batch to obtain PP fibers containing powdery sponge by a fusion spinning process. The amount of the master batch was adjusted such that the content of the sponge powder in the final fibers was 20 % by weight of the overall fibers.

Experiment example 18

Sponge powder was dispersed in an acrylic acid resin spun by the fusion spinning process while the resin was passing through an atmosphere at a temperature of fusing the resin surface, thus covering the fiber surface with sponge powder.

Comparative experiment examples 16 to 18

In Comparative experiment examples 16 to 18, polyester type polyurethane fibers, PP fibers and acrylic acid fibers which do not contain sponge powder were prepared in correspondence to Experiment examples 16 to 18, respectively.

Sensual test

The samples in Experiment examples 16 to 18 and Comparative experiment examples 16 to 18 are checked for the touch by sensual test by 10 persons. The result is shown in Table 15. It will be seen from the Figure that any experiment example sample has a sense of superior tough to that of the samples in the comparative experiment examples.

Table 15

| | Touch |
|---|---|
| Exp. example 16<br>Com. example 16 | O<br>Δ |
| Exp. example 17<br>Com. example 17 | O<br>Δ |
| Exp. example 18<br>Com. example 18 | ◎<br>Δ |

◎    O    Δ    ×
← Touch is good    Touch is bad →

The effect of the invention in connection with the fiber-finishing material was confirmed from an experiment example, in which the sponge powder prepared in Experiment example 1 was kneaded with a synthetic resin for the fiber-finishing material, and a comparative sample free from sponge powder.

Experiment example 19

An emulsion of an amino-denatured silicon type resin, the solid content of which can be formed into a film, was prepared by diluting 8.1 g of "Silicon AMZ" (manufactured by Nikka Kagaku Co., Ltd., synthetic resin content: 13 % by weight) with 338.g of added water. To this emulsion was added 13.5 g of the sponge powder prepared in Experiment example 1, and the system was stirred until it became homogenious. The mixture thus obtained was used to impregnate a nylon stocking material, and excess finishing material was removed using a squeezer comprising a metal roller and a rubber roller and with an inter-roll pressure of 1 kg/cm2. The resultant stocking material was dried in a drier at 120 °C for 5 minutes, thus obtaining a sample.

Comparative experiment example 19

The same nylon stocking material as in Experiment example 19 was not provided with any treatment.

Sensual test

The sense of touch was checked by sensual test by 10 persons, and also the moisture absorption and emission property was checked in the manner as in Experiment example 1. The result is shown in Fig. 16. It will be seen from the Table that the samples in the experiment examples are superior in the sense of touch and moisture absorption and emission properties.

Table 16

| | Touch | Moisture absorption and emission |
|---|---|---|
| Exp. example 19 | ◎ | O |
| Com. example 19 | × | × |

◎    O    Δ    ×
← Touch is good    Touch is bad →
 Moisture absorption    Moisture absorption
 and emission are high   and emission are low

The above individual experiment examples are by no means limitative, and the subjects in these examples may be increased, altered, improved or deformed without departing from the scope of the invention.

More specifically, the invention covers synthetic resins, rubber and all every other composition containing sponge in the form of powder and/or fibers, as well as products obtainable from these compositions and products, In which powdery or like sponge is incorporated by means of attachment. In general, the invention concerns any composition or product, which contains sponge powder or sponge fibers of a combination of such different forms of sponge.

According to the invention, by incorporating or attaching at least one member of the group consisting of sponge powder and sponge fibers in or to an artificial material, it is possible to obtain an anti-bacteria property and an ultra-violet cutting effect in addition to high moisture absorption and emission properties and good touch and appearance of manual materials.

## Claims

1. A sponge-containing composition containing 1 to 9 % by weight of at least one sponge material selected from the group consisting of sponge powder with an average grin size of, for instance, 0.1 to 100 μm and sponge fibers with a fiber length of 1 cm or below.

2. A sponge-containing composition according to claim 1, wherein the white degree of said sponge power or sponge fibers is 30 or above.

3. The sponge-containing composition according to claim 2, wherein said white degree is attained by a bleaching treatment at 50 °C or below.

4. The sponge-containing composition according to claim 3, wherein said bleaching treatment is carried out in three steps of oxidization bleaching, hydrogen peroxide breaching and reduction bleaching.

5. The sponge-containing composition according to claim 1, which contains at least one sponge material selected from said group and at least one material selected from the group consisting of synthetic resins and rubber.

6. A film or a sheet obtained from the sponge-containing composition according to claim 5.

7. A laminate comprising the film or sheet according to claim 6, said sheet or film being provided as a coating layer directly or via an adhesive on a base material.

8. A paint or an ink composed of at least one kind of resin and containing at least one sponge material selected from said group.

9. A cosmetic containing at least one sponge material selected from the group as set forth in claim 1.

10. Fibers obtained by obtaining a paste composition prepared by kneading together at least one sponge material selected from the group as set forth in claim 1 and at least one material selected from the group consisting of spinning material compounds for synthetic fibers, semi-synthetic fibers and regenerated fibers and spinning said paste composition.

11. A sponge-containing composition containing at least one sponge material selected from the group set forth in claim 1 and at least one material selected from the group consisting of synthetic resins for fiber-treating materials.

12. A sponge-containing product obtained by attaching at least one sponge material selected from the group as set forth in claim 1 to a base material.

13. A sponge-containing product obtained by preparing a paste composition by kneading one or more materials selected from the group consisting of spinning material compounds for synthetic fibers, semi-synthetic fibers and regenerated fibers, spinning said paste composition to obtain material fibers and covering the surfaces of said material fibers by attaching at least one sponge material selected from the group consisting of sponge powder and sponge fibers to said material fiber surfaces.

# F I G. I

# F I G. 2

# F I G. 3

# F I G. 4

# F I G. 5

FIG. 6

EP 0 551 762 A1

**European Patent Office**

**EUROPEAN SEARCH REPORT**

Application Number

EP 92 31 1772

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | Derwent Publications Ltd., London, GB; AN 77-63463Y & JP-A-52 047 897 (BRIDGESTONE TIRE) 16 April 1977 * abstract * --- | 1-13 | C08K11/00 C08L99/00 |
| X | Derwent Publications Ltd., London, GB; AN 76-02865X & JP-A-50 037 712 (KANEBO KK) 4 December 1975 * abstract * ----- | 1-13 | |
| | | | **TECHNICAL FIELDS SEARCHED (Int. Cl.5)** |
| | | | C08K C08L |

The present search report has been drawn up for all claims

| Place of search | Date of completion of the search | Examiner |
|---|---|---|
| THE HAGUE | 16 APRIL 1993 | LENTZ J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

30